(19) Europäisches Patentamt
European Patent Office
Office européen des brevets

(11) Veröffentlichungsnummer : **0 038 777**
**B1**

(12) # EUROPÄISCHE PATENTSCHRIFT

(45) Veröffentlichungstag der Patentschrift : **05.10.83**

(51) Int. Cl.³ : **C 07 C 46/00**, C 07 C 50/34

(21) Anmeldenummer : **81810143.8**

(22) Anmeldetag : **14.04.81**

(54) **Verfahren zur Herstellung von in 5- und 8-Stellung bisubstituierten OH- oder Chlorderivaten des Chinizarins.**

(30) Priorität : **22.04.80 CH 3106/80**
**22.04.80 CH 3107/80**

(43) Veröffentlichungstag der Anmeldung : **28.10.81 Patentblatt 81/43**

(45) Bekanntmachung des Hinweises auf die Patenterteilung : **05.10.83 Patentblatt 83/40**

(84) Benannte Vertragsstaaten : **BE CH DE FR GB IT LI**

(56) Entgegenhaltungen :
**EP A 0 007 051**
**BE A 651 989**
**DE A 2 758 397**
**GB A 1 357 955**
**US A 1 790 932**

(73) Patentinhaber : **CIBA-GEIGY AG**
**Patentabteilung Postfach**
**CH-4002 Basel (CH)**

(72) Erfinder : **Bloch, Peter, Dr.**
**Frobenstrasse 10**
**CH-4053 Basel (CH)**
Erfinder : **Adam, Jean-Marie, Dr.**
**3, Rue de Montreux**
**F-68300 Saint-Louis (FR)**

**EP 0 038 777 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Herstellung von in 5- und 8-Stellung bisubstituierten OH- oder Chlorderivaten des Chinizarins.

Gegenstand der Erfindung ist ein Eintopf-Verfahren zur Herstellung von 1,4,5,8-Tetrahydroxyanthrachinon, im weiteren Verlauf Dichinizarin genannt, oder von 5,8-Dichlor-1,4-dihydroxyanthrachinon, durch Reaktion von Phthalsäureanhydrid mit p-Chlorphenol in Oleum und in Gegenwart von Borkatalysatoren zum 1,4-Dihydroxyanthrachinonborkomplex und dessen direkte Weiterchlorierung mittels Chlorierungsmitteln im gleichen Reaktionsmedium zum 5,8-Dichlor-1,4-dihydroxyanthrachinonborkomplex sowie dessen Hydrolyse zu Dichinizarin.

Das neue Verfahren läuft nach folgendem Formelschema ab :

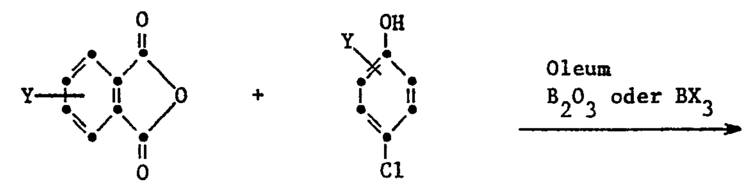


X = OH, Halogen, OAcyl,
Y = unabhängig voneinander Alkyl $C_1$-$C_4$, Halogen, vorzugsweise H,

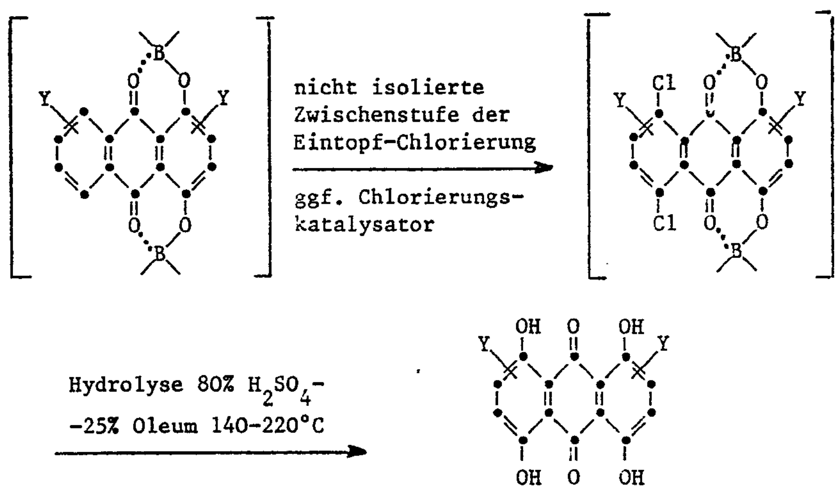


Der Substituent Y kann sich an p-Chlorphenol in o- oder m-Stellung zur OH-Gruppe befinden, am Phthalsäureanhydrid (1,2-Benzoldicarbonsäureanhydrid) in 4- oder 5-Stellung.

Die Herstellung von Chinizarin aus Phthalsäureanhydrid und p-Chlorphenol ist zwar bekannt, z. B. aus der DE-A 27 58 397.

Ebenfalls ist die Herstellung von 5,8-Dichlor-1,4-dihydroxyanthrachinon ausgehend von Chinizarin und dessen Chlorierung bekannt, z. B. aus der BE-A 651 989. Auch die Hydrolyse von 5,8-Dichlor-1,4-dihydroxyanthrachinon zu Dichinizarin ist bereits bekannt.

Die drei Verfahren wurden bisher jedoch getrennt betrieben, da die Säurekonzentrations- und Temperatur-Verhältnisse des Reaktionsmediums zur Erzielung reiner Produkte und guter Ausbeuten der einzelnen Stufen eine derart wichtige Rolle spielen, dass bei einer Kombination der drei Reaktionsstufen eine Lösung der Problematik einer ökologisch und ökonomisch besseren Gestaltung des Verfahrens als Eintopfreaktion nicht zu erwarten war.

Bei der Herstellung des Chinizarins durch Umsetzung von Phthalsäureanhydrid mit p-Chlorphenol in Schwefelsäure bzw. Oleum in Gegenwart von Borsäure fallen grosse Mengen an Abfallschwefelsäure an, deren Aufarbeitung durch Neutralisation und Beseitigung der dabei gebildeten grossen Salzmengen sowohl ökologische als auch ökonomische Schwierigkeiten bereiten. Ausserdem ist eine aufwendige Reinigung erforderlich, falls man die eingesetzte Borsäure erneut verwenden will.

Des weiteren erfolgt die Chlorierung des Chinizarins als Zwischenstufe ebenfalls in hochprozentiger Schwefelsäure oder Oleum in Gegenwart von Borsäure.

Während der nachfolgenden Aufarbeitung fällt wiederum verunreinigte Borsäure sowie eine grosse Menge Abfallschwefelsäure wegen der Verdünnung des Reaktionsansatzes an. Auch die Hydrolyse zu Dichinizarin verlangt eine grosse Menge Schwefelsäure.

Einerseits, durch die spezifischen reaktionskinetischen Erfordernisse bedingt, andererseits durch die geforderte hohe Reinheit des Zwischenproduktes Chinizarin gegeben, war man gezwungen, die jeweiligen Reaktionsstufen jede für sich auf optimale Reaktionsbedingungen auszurichten, was die vorher genannten, insbesondere ökologischen Nachteile verursachte. Es bestand deshalb ein echter

Bedarf an einem Eintopf-Verfahren, das die genannten Säurekonzentrations-Variationen umgeht und in dem die drei Reaktionsstufen im gleichen Reaktionsmedium durchgeführt werden können.

Es wurde nun gefunden, dass man nach der Umsetzung von Phthalsäureanhydrid mit p-Chlorphenol auf eine Isolierung und Reinigung des gebildeten Chinizarins verzichten kann und dass man die anschliessende Chlorierungsreaktion im Medium der hochprozentigen Schwefelsäure bzw. des Oleums direkt weiterführen und eine Hydrolyse zu Dichinizarin anschliessen kann. Ueberraschenderweise erhält man dabei 5,8-Dichlor-1,4-dihydroxyanthrachinon bzw. Dichinizarin in gleich guter Reinheit wie nach dem stufenweisen Verfahren, welches verschiedene zusätzliche Reinigungsoperationen beinhaltet. Ausserdem ist die Ausbeute an Endprodukten deutlich verbessert.

Das neue Verfahren zur Herstellung von, gegebenenfalls noch durch $C_1$-$C_4$-Alkyl- oder Halogen-substituiertem, 5,8-Dichlor- oder 5,8-Dihydroxychinizarin, ausgehend von Phthalsäureanhydrid und p-Chlorphenol, ist dadurch gekennzeichnet, dass man die Umsetzung von gegebenenfalls durch $C_1$-$C_4$-Alkyl oder Halogen substituiertem Phthalsäureanhydrid mit gegebenenfalls noch durch $C_1$-$C_4$-Alkyl oder Halogen substituiertem p-Chlorphenol in Oleum und in Gegenwart von $B_2O_3$ oder einer Verbindung $BX_3$, wobei X OH, Halogen oder OAcyl bedeutet, bei Temperaturen von 180 bis 220 °C kontinuierlich oder diskontinuierlich durchführt und den erhaltenen 1,4-Dihydroxyanthrachinon-Borkomplex, direkt ohne Isolierung, mittels Chlorierungsmitteln im gleichen Reaktionsmedium bei einer Temperatur zwischen 20 bis 150 °C chloriert und das erhaltene Produkt durch Verdünnen des sauren Mediums entweder bei einer Temperatur zwischen 70 bis 110 °C hydrolysiert und das entstehende 5,8-Dichlorchinizarin isoliert oder auf 80 %ige $H_2SO_4$ bis 25 %iges Oleum bei einer Temperatur zwischen 140 bis 220 °C zum 5,8-Dihydroxychinizarin hydrolysiert und dieses isoliert.

Das bevorzugte Verfahren zur Herstellung von 1,4,5,8-Tetrahydroxyanthrachinon ist dadurch gekennzeichnet, dass man die Umsetzung von p-Chlorphenol mit Phthalsäureanhydrid in Oleum in Gegenwart von $B_2O_3$, $H_3BO_3$ oder Borhalogenid bei Temperaturen von 180 bis 220 °C, vorteilhaft 200 bis 210 °C, kontinuierlich oder diskontinuierlich durchführt und den erhaltenen 1,4-Dihydroxy-anthrachinonborkomplex direkt, ohne dessen Isolierung, mittels üblichen Chlorierungsmitteln im gleichen Reaktionsmedium, gegebenenfalls unter Verwendung von Halogenierungskatalysatoren, bei einer Temperatur zwischen 20 bis 150 °C chloriert und das erhaltene Produkt durch Verdünnen des sauren Mediums auf 80 %ige $H_2SO_4$ bis 25 %iges Oleum bei einer Temperatur zwischen 140 bis 220 °C zu Dichinizarin, gegebenenfalls unter weiterem Borsäure-Zusatz, hydrolysiert und danach isoliert. Die Isolierung erfolgt in der Regel durch Herabsetzung der Oleumkonzentration.

Das bevorzugte Verfahren zur Herstellung von 5,8-Dichlor-1,4-dihydroxyanthrachinon durch Umsetzung von Phthalsäureanhydrid mit p-Chlorphenol ist dadurch gekennzeichnet, dass man die Umsetzung in Oleum und in Gegenwart von $B_2O_3$, $H_3BO_3$ oder Borhalogenid bei Temperaturen von 180 bis 220 °C kontinuierlich oder diskontinuierlich durchführt und den erhaltenen 1,4-Dihydroxyanthrachinon-Borkomplex, direkt ohne dessen Isolierung, mittels üblichen Chlorierungsmitteln im gleichen Reaktionsmedium bei einer Temperatur zwischen 20 bis 150 °C chloriert und das erhaltene Produkt durch Verdünnen des sauren Mediums bei einer Temperatur zwischen 70 bis 110 °C zu 5,8-Dichlor-1,4-dihydroxyanthrachinon hydrolysiert und isoliert.

Die beiden Ausgangsstoffe Phthalsäureanhydrid und p-Chlorphenol können in stöchiometrischen Mengen in die Reaktion eingesetzt werden. Es ist jedoch vorteilhaft, das billigere Phthalsäureanhydrid bis zum 1,5-fachen, insbesondere 1,3-fachen Ueberschuss, über die stöchiometrische Menge zu verwenden.

In der Regel wird die Umsetzung bei einem Gewichtsverhältnis von Oleum zu Phthalsäure von 1 : 1 bis 4 : 1, vorteilhaft 1,1 bis 1,5 : 1, bezogen auf 35 %iges Oleum, und von Bortrioxyd zu Phthalsäureanhydrid von 1 : 2 bis 1 : 4 durchgeführt. Die Konzentration des eingesetzten Oleums ist nicht kritisch und kann z. B. auch 70 % betragen.

Vor der Chlorierung wird das Reaktionsgemisch zweckmässig noch mit weiterem 50 bis 70 %igem Oleum und/oder Chlorsulfonsäure versetzt.

Geeignete Chlorierungsmittel sind z. B. Chlor, Chlorsulfonsäure, Thionylchlorid oder Sulfurylchlorid.

Bei Verwendung von Chlor arbeitet man gegebenenfalls unter leichtem Ueberdruck von bis zu 3 bar. Vorteilhaft wird die Chlorierung in Gegenwart von Halogenierungskatalysatoren durchgeführt.

Chlorsulfonsäure kann auch als Lösungsmittel in Gegenwart von Oleum eingesetzt werden.

Das neue Verfahren wird beispielsweise so durchgeführt, dass man in 35 %iges Oleum, Bortrioxid unter Rühren bei 100 bis 150 °C, vorteilhaft 120 bis 130 °C, einträgt. Anschliessend wird bei 130 bis 150 °C Phthalsäureanhydrid und dann p-Chlorphenol eingetragen, hierauf das Reaktionsgemisch unter guter Durchmischung auf 180 bis 220 °C, vorteilhaft 200 bis 210 °C, erhitzt und während 6 bis 15 Stunden bei dieser Reaktionstemperatur gehalten. Nach beendeter Zwischenreaktion wird auf 160 bis 170 °C abgekühlt und während des weiteren Abkühlens auf ca. 50 bis 120 °C wird weiteres 50 bis 70 %iges Oleum oder/und Chlorsulfonsäure in der mindestens doppelten Menge inbezug auf das am Anfang der Reaktion eingesetzte Oleum zugesetzt, um die Konsistenz des Reaktionsmediums im gewünschten Rahmen zu halten und gleichzeitig für die Chlorierung die günstigten Bedingungen zu schaffen.

Nach Zugabe von Halogenierungskatalysator, z. B. $SCl_2$, aber vorzugsweise Jod, wird bei einer Temperatur zwischen 20 bis 150 °C, vorzugsweise 50 bis 100 °C, mit Hilfe eines der vorher genannten Chlorierungsmittel chloriert, bis alles Chinizarin chloriert ist. Ueberschüssiges Chlor wird ausgeblasen und das Reaktionsgemisch mit Wasser, vorteilhaft auf eine Konzentration von 85 bis 95 % $H_2SO_4$

verdünnt. Anschliessend wird nochmals ca. 3 bis 15 Stunden auf 140 bis 220 °C erhitzt, wonach das entstandene Dichinizarin durch weiteres Verdünnen mit Wasser ausgefällt und isoliert wird.

Dabei ist es vorteilhaft, dem Gemisch noch geringe Mengen eines nicht-ionogenen Tensides zuzusetzen.

Der Zusatz des Tensids bewirkt eine bessere Kristallausbildung. Dadurch ist das Produkt besser handhabbar inbezug auf Filtration und Fliessverhalten der trockenen Ware.

Setzt man in die Reaktion Substrate mit im Reaktionsmedium stabilen Substituenten ein, so erhält man als Produkte entsprechend substituierte Dichinizarine.

Beispiele für derartige Reaktionssubstrate sind z. B. $C_1$-$C_4$-alkyliertes oder halogeniertes Phthalsäureanhydrid und/oder p-Chlorphenol. Das neue Eintopf-Verfahren ist viel effizienter als die bisher angewandten einzelnen Stufenverfahren.

So werden z. B. gemäss dem neuen Verfahren Ausbeuten von über 90 % (berechnet auf p-Chlorphenol) von Produkten von guter Qualität erhalten, wogegen die Summe der Ausbeuten der einzelnen Stufen des stufenweisen Vorgehens bei nur höchstens 80 % liegt. Darüberhinaus entfallen folgende bisher nach dem Stufenverfahren übliche Verfahrensschritte :

— Austragen der Zwischenstufe des Chinizarin-Borkomplexes auf Eis oder Wasser ;

— Hydrolyse des Chinizarin-Borkomplexes zu Chinizarin ;

— Herstellen einer Lösung des Chinizarin-Borkomplexes in Oleum zur zweiten Umsetzungsstufe, Aufheizen der Lösung auf Chlorierungstemperatur.

— Herstellung des Dichlor-chinizarin-borkomplexes in Schwefelsäure

— Filtrieren, Waschen und Trocknen des Chinizarins und Dichlorchinizarin

— Aufbereiten der borsäure- und schwefelsäurehaltigen Abwässer der ersten beiden Stufen.

Der Wegfall obiger Operationen führt zu erheblichen ökologischen und ökonomischen Vorteilen. Die wichtigsten davon sind : man erhält

— ca. 50 % weniger Borverbindungen im Abwasser ;

— je nach Vergleichsverfahren bis über 70 % weniger Schwefelsäure im Abwasser ;

Es wird ein geringerer Aufwand an Zeit, Personal und Apparaten sowie an Energie benötigt, was natürlich eine kostengünstigere Verfahrensgestaltung zur Folge hat.

Nach dem erfindungsgemässen Verfahren hergestelltes Dichlorchinizarin und Dichinizarin lassen sich ohne weitere Reinigung zu Farbstoffen umsetzen, z. B. durch Kondensation mit Aminen.

Folgende Beispiele veranschaulichen das neue Verfahren, ohne es auf diese zu beschränken.

Teile bedeuten Gewichtsteile, falls nicht anders formuliert.

## Beispiel 1

In einem 500 ml Kolben werden 77 Teile Schwefelsäuremonohydrat vorgelegt und 89 Teile Oleum 66 % zugegeben, wobei die Temperatur bis auf 45 bis 50° steigt. Daraufhin werden 38 Teile Bortrioxid unter Rühren so eingetragen, dass die Temperatur auf 120 bis 125 °C steigt. Dafür werden ca. 20 Minuten benötigt. Es entsteht eine milchige Lösung. Der Kolbeninhalt wird auf 150 °C aufgeheizt und es werden dann bei 130 bis 150 °C zuerst 100 Teile Phthalsäureanhydrid und anschliessend 64 Teile p-Chlorphenol eingetragen. Hierauf wird das Reaktionsgemisch auf 205 ± 2 °C aufgeheizt und während 14 Stunden bei dieser Temperatur gehalten.

Es wird nun auf 160 bis 170 °C abgekühlt. Während des weiteren Abkühlens bis 100 °C werden 40 Teile Oleum 66 % langsam zugetropft, um gute Rührbarkeit zu gewährleisten. Weitere 325 Teile Oleum 66 % werden bei 70 °C — zunächst unter Kühlen — zugetropft. Nach Zugabe von 5 Teilen Jod (fein pulverisiert) wird bei 65 bis 70 °C so lange elementares Chlor eingeleitet, bis die chromatographische Prüfung einer entnommenen Probe kein Chinizarin mehr erkennen lässt.

Das überschüssige Chlor wird aus dem Reaktionsgemisch durch Luft oder Stickstoff vertrieben. Dann wird die Schwefelsäurekonzentration, durch Verdünnung mit Wasser, auf 90 % gestellt und das Reaktionsgemisch bei 180 °C gerührt, bis sich chromatographisch kein Dichlorchinizarin mehr nachweisen lässt. Die Lösung wird in 200 Teile wässrige ca 6 %ige Natriumbisulfit-Lösung ausgetragen, mit 200 Teilen eines handelsüblichen Tensids auf Polyetherbasis versetzt und 1 bis 3 Stunden bei 85 bis 90 °C gerührt. Hierauf wird filtriert, heiss neutral gewaschen und getrocknet. Man erhält 125 Teile 1,4,5,8-Tetrahydroxy-anthrachinon = 91,8 % der theoretischen Ausbeute, bezogen auf eingesetztes Chlorphenol.

Ein nach diesem Verfahren hergestelltes Reaktionsgemisch wird wie oben ausgefällt, aber ohne Tensidzusatz. Das Produkt ist deutlich schlechter filtrierbar.

## Beispiel 1a : Variante zu 5,8-Dichlorchinizarin

Nach der Chlorierung wie in Beispiel 1, wird das überschüssige Chlor durch Einleiten von Stickstoff aus dem Reaktionsgemisch vertrieben, letzteres in 2 000 Teile wässrige ca. 6 %ige Natriumbisulfit-Lösung ausgetragen, mit 20 Teilen eines handelsüblichen Tensids auf Polyetherbasis versetzt und 2 Stunden bei 85 bis 90 °C gerührt. Hierauf wird filtriert, heiss neutral gewaschen und getrocknet.

Ausbeute : 145,3 Teile 5,8-Dichlorchinizarin = 94 % der Theorie, bezogen auf Chlorphenol.

Chlorgehalt : 22,9 % (theoretisch 22,94 %).

### Vergleichsbeispiel

Ein nach dem Verfahren von Beispiel 1 hergestelltes Reaktionsgemisch wird wie oben auf wässrige Bisulfitlösung ausgetragen und gleich lange wie in Beispiel 1, aber ohne Tensid, bei 85 bis 90 °C gerührt. Das Produkt ist deutlich schlechter filtrierbar als in Beispiel 1 und weist bei etwas schlechterer Ausbeute einen Chlorgehalt von 24,5 % auf.

### Beispiel 2

38 Teile Bortrioxid werden in 166 Teile Oleum 66 % eingetragen und vorsichtig (exotherme Reaktion) auf 150 °C geheizt. Darauf werden 100 Teile Phthalsäureanhydrid und 64,3 Teile Chlorphenol eingetragen, das Gemisch auf 205 °C aufgeheizt und 14 Stunden gerührt. Man kühlt auf 160 °C und hält das Gemisch während des weiteren Abkühlens bis 90 °C durch Zutropfen von 40 Teilen Oleum 66 % rührbar. Dann werden 400 Teile Oleum 66 %, 5 Teile Jod und anschliessend 550 Teile Chlorsulfonsäure langsam zugegeben. Man rührt hierauf bei 90 bis 100 °C, bis sich eine entnommene Probe als Chinizarin-frei erweist. Das Reaktionsgemisch lässt man langsam in 3 000 Teile Eiswasser laufen, dem vorher 150 Teile 40 %iges Bisulfitlösung und 20 Teile Tensid zugesetzt wurden, und kocht anschliessend 3 Stunden unter Rückfluss. Nach Filtrieren, Waschen und Trocknen erhält man 5,8-Dichlorchinizarin in gleich guter Ausbeute wie in Beispiel 1a.

### Beispiel 3

In 435 Teile Oleum 9 % werden nacheinander 0,02 Teile Nitrobenzolsulfonsäure, 42,9 Teile $H_3BO_3$, 97,9 Teile Phthalsäureanhydrid und, wenn die Lösung klar geworden ist, 64,3 Teile Chlorphenol eingetragen. Die Mischung wird innerhalb 5 Stunden auf 195 °C erhitzt und 10 Stunden bei dieser Temperatur gehalten. Dann wird auf 65 °C gekühlt und, nach Zusatz von 5 Teilen Jod und 800 Teilen Oleum 66 %, bei 65 bis 70 °C Chlor eingeleitet. Nach beendeter Chlorierung wird wie in Beispiel 1 aufgearbeitet. Es werden 126 Teile 5,8-Dichlorchinizarin erhalten.

### Beispiel 4

In 200 Teile Oleum 20 % werden zuerst 38 Teile Bortrioxid und dann bei 120 °C 100 Teile Phthalsäureanhydrid und 64,3 Teile Chlorphenol eingetragen. Das Gemisch wird 16 Stunden auf 200 °C erhitzt, auf 65 °C gekühlt, mit 400 Teilen Oleum 66 % und 5 Teilen Jod versetzt und dann Chlor eingeleitet. Nach beendeter Chlorierung wird wie in Beispiel 1 aufgearbeitet. Es werden 128 Teile 5,8-Dichlorchinizarin erhalten.

### Beispiel 5 und 6

Man arbeitet wie in Beispiel 2, verwendet aber als Chlorierungsmittel, statt Chlorsulfonsäure, Thionylchlorid bzw. Sulfurylchlorid in den benötigten Mengen. Es werden ähnliche Ausbeuten an gleichwertigen Endprodukten wie in Beispiel 1a erhalten.

### Beispiel 7

Man arbeitet wie im Beispiel 1, nach der Chlorierung wird aber die Schwefelsäurekonzentration auf 95 % gestellt und das Reaktionsgemisch bei 200° gerührt, bis kein Ausgangsmaterial mehr nachweisbar ist. 1,4,5,8-Tetrahydroxy-anthrachinon wird in gleich guter Ausbeute erhalten wie im Beispiel 1.

### Beispiel 8

Verfährt man wie im Beispiel 7 und hydrolysiert das 5,8-Dichlorchinizarin unter Zusatz von 25 Teilen Borsäure, so erhält man 1,4,5,8-Tetrahydroxy-anthrachinon in guter Ausbeute in einer um die Hälfte kürzeren Reaktionszeit.

**Ansprüche**

1. Verfahren zur Herstellung von, gegebenenfalls noch durch $C_1$-$C_4$-Alkyl oder Halogen substituiertem, 5,8-Dichlor- bzw. 5,8-Dihydroxychinizarin, ausgehend von Phthalsäureanhydrid und p-Chlorphenol, dadurch gekennzeichnet, dass man die Umsetzung von gegebenenfalls durch $C_1$-$C_4$-Alkyl oder Halogen substituiertem Phthalsäureanhydrid mit gegebenenfalls noch durch $C_1$-$C_4$-Alkyl oder Halogen substituiertem p-Chlorphenol in Oleum und in Gegenwart von $B_2O_3$ oder einer Verbindung $BX_3$, wobei X

OH, Halogen oder -O-Acyl bedeutet, bei Temperaturen bon 180 bis 220 °C kontinuierlich oder diskontinuierlich durchführt und den erhaltenen 1,4-Dihydroxyanthrachinon-Borkomplex, direkt ohne dessen Isolierung, mittels Chlorierungsmitteln im gleichen Reaktionsmedium bei einer Temperatur zwischen 20 bis 150 °C chloriert und das erhaltene Produkt durch Verdünnen des sauren Mediums entweder bei einer Temperatur zwischen 70 bis 110 °C hydrolysiert und das entstehende 5,8-Dichlorchinizarin isoliert oder auf 80 %ige $H_2SO_4$ bis 25 %iges Oleum bei einer Temperatur zwischen 140 bis 220 °C zum 5,8-Dihydroxychinizarin hydrolysiert und dieses isoliert.

2. Verfahren zur Herstellung von 1,4,5,8-Tetrahydroxyanthrachinon, ausgehend von Phthalsäureanhydrid und p-Chlorphenol, gemäß Anspruch 1, dadurch gekennzeichnet, dass man die Umsetzung in Oleum und in Gegenwart von $B_2O_3$, $H_3BO_3$ oder Borhalogenid bei Temperaturen von 180 bis 220 °C kontinuierlich oder diskontinuierlich durchführt und den erhaltenen 1,4-Dihydroxyanthrachinon-Borkomplex, direkt ohne dessen Isolierung, mittels üblichen Chlorierungsmitteln im gleichen Reaktionsmedium bei einer Temperatur zwischen 20 bis 150 °C chloriert und das erhaltene Produkt durch Verdünnen des sauren Mediums auf 80 %ige $H_2SO_4$ bis 25 %iges Oleum bei einer Temperatur zwischen 140 bis 220 °C zu 5,8-Dihydroxychinizarin hydrolysiert und dieses isoliert.

3. Verfahren zur Herstellung von 5,8-Dichlor-1,4-dihydroxyanthrachinon ausgehend von Phthalsäureanhydrid mit p-Chlorphenol gemäß Anspruch 1, dadurch gekennzeichnet, dass man die Umsetzung in Oleum und in Gegenwart von $B_2O_3$, $H_3BO_3$ oder Borhalogenid bei Temperaturen von 180 bis 220 °C kontinuierlich oder diskontinuierlich durchführt und den erhaltenen 1,4-Dihydroxyanthrachinon-Borkomplex, direkt ohne dessen Isolierung, mittels üblichen Chlorierungsmitteln im gleichen Reaktionsmedium bei einer Temperatur zwischen 20 bis 150 °C chloriert und das erhaltene Produkt durch Verdünnen des sauren Mediums bei einer Temperatur zwischen 70 und 110 °C zu 5,8-Dichlorchinizarin hydrolysiert und isoliert.

4. Verfahren gemäss Ansprüchen 1-3, dadurch gekennzeichnet, dass man dem Reaktionsmedium vor der Chlorierung soviel weiteres 50 bis 70 %iges Oleum und/oder Chlorsulfonsäure zusetzt, dass es mindestens die doppelte Menge des am Anfang der Reaktion eingesetzten Oleums ist.

5. Verfahren gemäss Anspruch 1, dadurch gekennzeichnet, dass man die Kondensationsreaktion bei 200 bis 210 °C und die Chlorierungsreaktion bei 50 bis 100 °C durchführt.

6. Verfahren gemäss Anspruch 1, dadurch gekennzeichnet, dass man p-Chlorphenol und Phthalsäureanhydrid im stöchiometrischen Verhältnis von 1 : 1 bis 1 : 1,5 verwendet.

7. Verfahren gemäss Ansprüchen 1-4, dadurch gekennzeichnet, dass man Oleum und Phthalsäureanhydrid in der ersten Stufe im Gewichtsverhältnis von 1 : 1 bis 4 : 1 verwendet.

8. Verfahren gemäss Ansprüchen 1-3, dadurch gekennzeichnet, dass man Bortrioxid und Phthalsäureanhydrid im Gewichtsverhältnis von 1 : 2 bis 1 : 4 verwendet.

9. Verfahren gemäss Anspruch 1, dadurch gekennzeichnet, dass man die Chlorierung in Gegenwart eines Halogenierungskatalysators durchführt.

10. Verfahren gemäss Anspruch 1, dadurch gekennzeichnet, dass man Chlor als Chlorierungsmittel verwendet.

11. Verfahren gemäss Anspruch 1, dadurch gekennzeichnet, dass man zum verdünnten sauren Medium ein nichtionogenes Tensid hinzusetzt.

**Claims**

1. A process for the production of 5,8-dichloro- or 5,8-dihydroxyquinizarine, each of which may be further substituted by $C_1$-$C_4$ alkyl or halogen, starting from phthalic anhydride and p-chlorophenol, which process comprises carrying out the reaction of phthalic anhydride which may be substituted by $C_1$-$C_4$ alkyl or halogen, with p-chlorophenol which may be further substituted by $C_1$-$C_4$ alkyl or halogen, in oleum and in the presence of $B_2O_3$ or a compound $BX_3$, wherein X is OH, halogen or -O-acyl, in the temperature range from 180° to 220 °C, either continuously or discontinuously, and chlorinating the boron complex of 1,4-dihydroxyanthraquinone obtained as intermediate, direct without isolation, with a chlorinating agent, in the same reaction medium and in the temperature range from 20° to 150 °C, and either hydrolysing the chlorinated product by diluting the acid medium in the temperature range from 70° to 110 °C and isolating the 5,8-dichloroquinizarine thereby obtained, or by diluting the acid medium to a concentration of 80 % $H_2SO_4$ to 25 % oleum, in the temperature range from 140° to 220 °C, and isolating the 5,8-dihydroxyquinizarine thereby obtained.

2. A process according to claim 1 for the production of 1,4,5,8-tetrahydroxyanthraquinone starting from phthalic anhydride and p-chlorophenol, which process comprises carrying out the reaction in oleum and in the presence of $B_2O_3$, $H_3BO_3$ or boron halide, in the temperature range from 180° to 220 °C, either continuously or discontinuously, and chlorinating the boron complex of 1,4-dihydroxyanthraquinone obtained as intermediate, direct without isolation, with a conventional chlorinating agent and in the same reaction medium, in the temperature range from 20° to 150 °C, and hydrolysing the chlorinated product by diluting the acid medium to a concentration of 80 % $H_2SO_4$ to 25 % oleum, in the temperature range from 140° to 220 °C, to 5,8-dihydroxyquinizarine, and isolating the hydrolysed product.

3. A process according to claim 1 for the production of 5,8-dichloro-1,4-dihydroxyanthraquinone

starting from phthalic anhydride and p-chlorophenol, which process comprises carrying out the reaction in oleum and in the presence of $B_2O_3$, $H_3BO_3$ or boron halide, in the temperature range from 180° to 220 °C, either continuously or discontinuously, and chlorinating the boron complex of 1,4-dihydroxyanthraquinone obtained as intermediate, direct without isolation, with a conventional chlorinating agent, in the same reaction medium and in the temperature range from 20° to 150 °C, and hydrolysing the chlorinated product to 5,8-dichloroquinizarine by diluting the acid medium in the temperature range from 70° to 110 °C, and isolating the hydrolysed product.

4. A process according to any one of claims 1 to 3, wherein 50 to 70 % oleum and/or chlorosulfonic acid is added to the reaction medium, before the chlorination, in an amount at least twice that of the oleum employed at the start of the reaction.

5. A process according to claim 1, wherein the condensation is carried out in the temperature range from 200° to 210 °C and the chlorination in the range from 50° to 100 °C.

6. A process according to claim 1, wherein p-chlorophenol and phthalic anhydride are used in the stoichiometric ratio of 1 : 1 to 1 : 1.5.

7. A process according to any one of claims 1 to 4, wherein oleum and phthalic anhydride are used in the first step in the weight ratio of 1 : 1 to 4 : 1.

8. A process according to any one of claims 1 to 3, wherein boron trioxide and phthalic anhydride are used in the weight ratio of 1 : 2 to 1 : 4.

9. A process according to claim 1, wherein the chlorination is carried out in the presence of a halogenating catalyst.

10. A process according to claim 1, wherein chlorine is used as chlorinating agent.

11. A process according to claim 1, wherein a non-ionic surfactant is added to the dilute acid medium.

## Revendications

1. Procédé pour la préparation de 5,8-dichloroquinizarine ou de 5,8-dihydroxyquinizarine substituées encore par un groupe alkyle en $C_1$-$C_4$ ou par de l'halogène, partant de l'anhydride phtalique et du p-chlorophénol, caractérisé par le fait qu'on effectue la réaction de l'anhydride phtalique éventuellement substitué par un groupe alkyle en $C_1$-$C_4$ ou par de l'halogène, avec du p-chlorophénol éventuellement substitué encore par un groupe alkyle en $C_1$-$C_4$ ou de l'halogène, dans l'Oléum et en présence de $B_2O_3$ ou d'un composé $BX_3$, où X est OH, un halogène ou un groupe -O-acyle, à des températures de 180 à 220 °C, d'une façon continue ou discontinue, et qu'on chlore le complexe borique de la 1,4-dihydroxyanthraquinone obtenu, directement sans le séparer, à l'aide d'agents de chloration dans le même milieu réactionnel, à une température comprise entre 20 et 150 °C et soit qu'on hydrolyse le produit obtenu par dilution du milieu acide, à une température comprise entre 70 et 110 °C et qu'on isole la 5,8-dichloroquinizarine formée, soit qu'on hydrolyse le produit obtenu par dilution du milieu acide, à une concentration en $H_2SO_4$ de 80 % jusqu'à une concentration en Oléum de 25 %, à une température comprise entre 140 et 220 °C, pour avoir la m-5,8-dihydroxyquinizarine et qu'on isole celle-ci.

2. Procédé pour la préparation de la 1,4,5,8-tétrahydroxyanthraquinone, partant de l'anhydride phtalique et du p-chlorophénol selon la revendication 1, caractérisé par le fait qu'on effectue en continu ou en discontinu la réaction dans l'Oléum et en présence de $B_2O_3$, $H_3BO_3$ ou d'halogénure de bore, à des températures de 180 à 220 °C, et qu'on chlore le complexe borique de la 1,4-dihydroxyanthraquinone obtenu, directement sans l'isoler, à l'aide d'agents usuels de chloration, dans le même milieu réactionnel, à une température comprise entre 20 et 150 °C, et qu'on hydrolyse le produit obtenu par dilution du milieu acide à une concentration en $H_2SO_4$ de 80 % jusqu'à une concentration en Oléum de 25 %, à une température comprise entre 140 et 220 °C, pour avoir la 5,8-dihydroxyquinizarine et qu'on isole celle-ci.

3. Procédé pour la préparation de la 5,8-dichloro-1,4-dihydroxyanthraquinone, partant de l'anhydride phtalique et du p-chlorophénol selon la revendication 1, caractérisé par le fait qu'on effectue en continu ou en discontinu la réaction dans l'Oléum et en présence de $H_2O_3$, $H_3BO_3$ ou d'halogénure de bore, à des températures de 180 à 220 °C, et qu'on chlore le complexe borique de la 1,4-dihydroxyanthraquinone obtenu, directement sans l'isoler, à l'aide d'agents usuels de chloration, dans le même milieu réactionnel, à une température comprise entre 20 et 150 °C, puis qu'on hydrolyse le produit obtenu par dilution du milieu acide à une température comprise entre 70 et 110 °C, pour avoir la 5,8-dichloroquinizarine et qu'on isole celle-ci.

4. Procédé selon les revendications 1 à 3, caractérisé par le fait qu'on ajoute au milieu réactionnel avant la chloration suffisamment d'Oléum supplémentaire à 50-70 % et/ou de chlorhydrine sulfurique pour que la quantité d'Oléum soit au moins le double de la quantité d'Oléum utilisé au début de la réaction.

5. Procédé selon la revendication 1, caractérisé par le fait qu'on effectue la réaction de condensation à 200-210 °C et la réaction de chloration à 50-100 °C.

6. Procédé selon la revendication 1, caractérisé par le fait qu'on utilise le p-chlorophénol et l'anhydride phtalique dans un rapport stœchiométrique de 1 : 1 à 1 : 1,5.

7. Procédé selon les revendications 1 à 4, caractérisé par le fait qu'on utilise l'Oléum et l'anhydride phtalique dans le premier stade dans le rapport pondéral de 1 : 1 à 4 : 1.

8. Procédé selon les revendications 1 à 3, caractérisé par le fait qu'on utilise le trioxyde de bore et l'anhydride phtalique dans le rapport pondéral de 1 : 2 à 1 : 4.

9. Procédé selon la revendication 1, caractérisé par le fait qu'on effectue la chloration en présence d'un catalyseur d'halogénation.

10. Procédé selon la revendication 1, caractérisé par le fait qu'on utilise le chlore comme agent de chloration.

11. Procédé selon la revendication 1, caractérisé par le fait qu'on ajoute un tensio-actif non ionique au milieu acide dilué.